(19) Europäisches Patentamt

European Patent Office

Office européen des brevets

(11) **EP 1 637 105 A1**

(12) **EUROPEAN PATENT APPLICATION**
published in accordance with Art. 158(3) EPC

(43) Date of publication:
22.03.2006 Bulletin 2006/12

(51) Int Cl.:
*A61F 13/53* (2000.01)   *A61L 15/60* (1990.01)
*B01J 20/26* (1980.01)

(21) Application number: 04745929.2

(22) Date of filing: 09.06.2004

(86) International application number:
PCT/JP2004/008377

(87) International publication number:
WO 2004/110328 (23.12.2004 Gazette 2004/52)

(84) Designated Contracting States:
AT BE BG CH CY CZ DE DK EE ES FI FR GB GR
HU IE IT LI LU MC NL PL PT RO SE SI SK TR

(30) Priority: 13.06.2003 JP 2003169595

(71) Applicant: SUMITOMO SEIKA CHEMICALS CO.,
LTD.
Kako-gun, Hyogo 675-0145 (JP)

(72) Inventors:
• YOSHINO, Kazuhiro
  Fukuoka 818-0031 (JP)
• KAWAKITA, Tomoki
  Shikama-ku, Himeji-shi, Hyogo 672-8076 (JP)
• NAWATA, Yashuhiro
  Shikama-ku, Himeji-shi, Hyogo 672-8076 (JP)

(74) Representative: Vossius & Partner
Siebertstrasse 4
81675 München (DE)

(54) **ABSORBING MATERIAL AND ABSORPTIVE ARTICLE USING THE SAME**

(57) An absorbent comprising a water-absorbent resin and a hydrophilic fiber, **characterized in that** the above-mentioned water-absorbent resin has a water absorption (a) of physiological saline of 60 to 100 g/g and a water-retaining capacity (b) of physiological saline of 45 to 80 g/g, and that the water absorption (a) of physiological saline and the water-retaining capacity (b) of physiological saline satisfy the relationship of the formula: [Water absorption (a) of physiological saline] ≥ [Water-retaining capacity (b) of physiological saline] + 15, and an absorbent article, wherein the above-mentioned absorbent is interposed between a liquid-permeable sheet and a liquid-impermeable sheet.

# FIG. 1

EP 1 637 105 A1

**Description**

TECHNICAL FIELD

The present invention relates to an absorbent and an absorbent article in which the absorbent is used. More specifically, the present invention relates to an absorbent which can be suitably used in hygienic materials such as disposable diapers, incontinence pads and sanitary napkins, and an absorbent article in which the absorbent is used.

BACKGROUND ART

Absorbent articles such as disposable diapers, sanitary napkins, incontinence pads and breast milk pads have a structure in which an absorbent is interposed between a liquid-permeable sheet to be placed on a side contacting a body and a liquid-impermeable sheet to be placed on a side opposite thereto. In addition, an absorbent has an action of absorbing and retaining an aqueous liquid such as urine or blood excreted from a body, and comprises a water-absorbent resin which absorbs and retains an aqueous liquid, and a hydrophilic fiber.
In recent years, progress has been made in thinning absorbent articles. In order not to lower water absorption in the absorbent even when subjected to thinning, the amount of a bulky hydrophilic fiber having a low water absorption and a low water-retaining capacity is decreased, and the amount of a water-absorbent resin having a high water absorption and a high water-retaining capacity is increased. As the absorbent and the absorbent article in which the absorbent is used, for example, an absorbent using a water-absorbent resin of which specified characteristics have been remarked and an absorbent article in which the absorbent is used have been known (see, for example, U.S. Patent No. 5,147,343, Japanese Patent Laid-Open No. Hei 5-200068, Japanese Patent Laid-Open No. Hei 6-254118 and Japanese Unexamined Patent Publication No. Hei 9-510889).
However, the above-mentioned absorbent and the absorbent article in which the absorbent is used are not fully satisfactory from the viewpoint of liquid leakage during absorption and re-wet after the absorption. In addition, there are some problems such that the amount of a water-absorbent resin is increased in the absorbent, so that the absorbent is expensive and has an increased heavy feel, and a harder texture.
Therefore, in recent years, there has been desired the development of an absorbent capable of being thinned, which has a small amount of re-wet of an aqueous liquid and a high permeation rate and is excellent in diffusibility, without increasing the amount of the water-absorbent resin in the absorbent.

DISCLOSURE OF INVENTION

An object of the present invention is to provide an absorbent having a small amount of re-wet of an aqueous liquid and a high permeation rate and being excellent in diffusibility, without increasing the amount of the water-absorbent resin in the absorbent, and an absorbent article in which the absorbent is used.
Specifically, the present invention relates to an absorbent comprising a water-absorbent resin and a hydrophilic fiber, characterized in that the above-mentioned water-absorbent resin has a water absorption (a) of physiological saline of 60 to 100 g/g and a water-retaining capacity (b) of physiological saline of 45 to 80 g/g, and that the water absorption (a) of physiological saline and the water-retaining capacity (b) of physiological saline satisfy the relationship of the formula:

$$[\text{Water absorption (a) of physiological saline}] \geq [\text{Water-retaining capacity (b) of physiological saline}] + 15$$

The water-absorbent resin having a water absorption (a) of physiological saline of 100 g/g and a water-retaining capacity (b) of physiological saline of 80 g/g can be obtained by a reaction in which an azo-radical polymerization initiator having a low self-crosslinking reactivity is used as a radical polymerization initiator, and a chain transfer agent is added thereto during the polymerization reaction. In addition, a water-absorbent resin having a water absorption (a) of physiological saline of 60 g/g and a water-retaining capacity (b) of physiological saline of 45 g/g can be obtained by a reaction in which a radical polymerization initiator other than the azo-radical polymerization initiator is used, and a chain transfer agent and/or a metal is added during the polymerization reaction.

BRIEF DESCRIPTION OF THE DRAWINGS

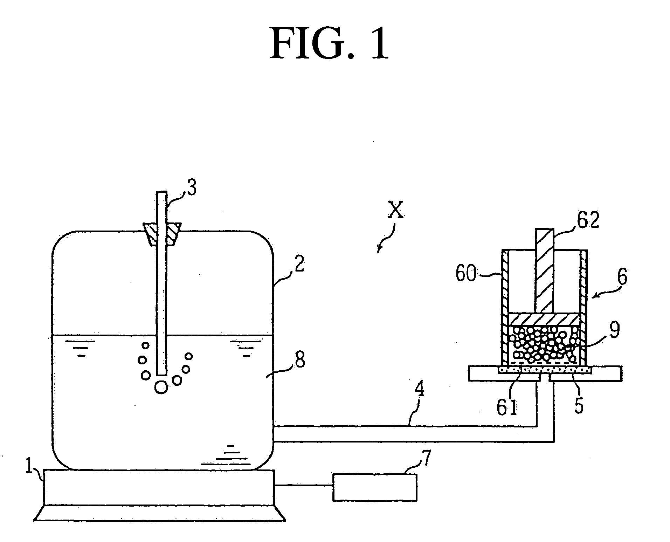

Figure 1 is a schematic explanatory view of an apparatus for determining the water absorption under pressure.

BEST MODE FOR CARRYING OUT THE INVENTION

The absorbent and the absorbent article in which the absorbent of the present invention is used will be explained hereinbelow.

The absorbent article includes, for example, disposable hygienic materials such as disposable diapers, incontinence pads, sanitary napkins, breast milk pads and the like. The present invention is not limited only to those exemplified ones.

The absorbent article of the present invention has a structure in which an absorbent for absorbing and retaining an aqueous liquid is interposed between a liquid-permeable sheet (top sheet) which is capable of passing the aqueous liquid therethrough and a liquid-impermeable sheet (back sheet) which is incapable of passing the aqueous liquid to pass therethrough. The liquid-permeable sheet is placed on a side contacting a body and the liquid-impermeable sheet is placed on a side not contacting the body.

The liquid-permeable sheet includes, for example, nonwoven fabrics made of polyethylene, polypropylene, polyester, polyamide or the like; porous synthetic resin sheets; and the like, and the present invention is not limited only to those exemplified ones.

The liquid-impermeable sheet includes, for example, films made of a synthetic resin such as polyethylene, polypropylene or polyvinyl chloride; sheets made of composite materials comprising a film made of a synthetic resin mentioned above and a nonwoven fabric; and the like, and the present invention is not limited only to those exemplified ones.

The size of the liquid-permeable sheet and the liquid-impermeable sheet cannot be absolutely determined since the size differs depending upon the applications of the absorbent articles. Therefore, it is preferable that the size is appropriately adjusted in accordance with its purposes.

The basis weight of the liquid-permeable sheet and the liquid-impermeable sheet cannot be absolutely determined since the basis weight differs depending upon the applications of the absorbent articles. For example, when the absorbent articles are disposable diapers, usually, each of their basis weight is preferably 10 to 300 $g/m^2$, and more preferably 15 to 200 $g/m^2$.

The absorbent of the present invention comprises a water-absorbent resin and a hydrophilic fiber. The content of the water-absorbent resin in the absorbent is preferably at least 5% by weight and less than 60% by weight, and more preferably 10 to 50% by weight from the viewpoint of thinning of the absorbent.

The hydrophilic fiber includes, for example, cellulose fibers such as cotton pulp, mechanical pulp, chemical pulp and semi-chemical pulp; artificial cellulose fibers such as rayon and acetate; and the like. The hydrophilic fiber may contain a synthetic fiber such as a polyamide, a polyester or a polyolefin, and the hydrophilic fiber is not limited thereto.

The constitution of the absorbent includes, for example, a mix structure in which a water-absorbent resin and a hydrophilic fiber are homogeneously blended; a sandwich structure in which a water-absorbent resin is interposed between layers of hydrophilic fibers; a structure in which a water-absorbent resin and a hydrophilic fiber are wrapped with tissue paper; and the like, and the present invention is not limited only to those exemplified ones. The absorbent of the present invention may contain a synthetic fiber as a reinforcing material.

The water absorption (a) of physiological saline by the water-absorbent resin is at least 60 g/g, and preferably at least 65 g/g from the viewpoint of lowering the amount of re-wet of the resulting absorbent. The water absorption (a) is at most 100 g/g, and preferably at most 90 g/g from the viewpoint of fortifying gel strength, thereby allowing to diffuse an aqueous liquid when the water-absorbent resin is used in a thin absorbent, and lowering the amount of re-wet. From these viewpoints, the water absorption (a) of physiological saline of the water-absorbent resin is 60 to 100 g/g, and preferably 65 to 90 g/g.

Incidentally, the water-absorbent resin having a highest water absorption (a) of physiological saline can be obtained by a reaction in which an azo-radical polymerization initiator having a low self-crosslinking reactivity is used as a radical polymerization initiator, and a chain transfer agent is added thereto during the polymerization reaction. In addition, the water-absorbent resin having a lowest water absorption (a) of physiological saline can be obtained by a reaction in which a radical polymerization initiator other than the azo-radical polymerization initiator is used, and a chain transfer agent and/or a metal is added thereto during the polymerization reaction.

The water absorption (a) of physiological saline of the water-absorbent resin is a value obtained in accordance with the determination method described in "(1) Water Absorption of Physiological Saline" given later.

The water-retaining capacity (b) of physiological saline of the water-absorbent resin is at least 45 g/g, and preferably at least 50 g/g from the viewpoint of lowering the amount of re-wet of the absorbent. The water-retaining capacity (b) is at most 80 g/g, and preferably at most 70 g/g from the viewpoint of fortifying gel strength, thereby allowing to diffuse an aqueous liquid when the water-absorbent resin is used in a thin absorbent, and lowering the amount of re-wet. From these viewpoints, the water-retaining capacity (b) of physiological saline of the water-absorbent resin is 45 to 80 g/g, preferably 45 to 70 g/g, and more preferably 50 to 70 g/g.

The water-absorbent resin having a highest water-retaining capacity (b) of physiological saline can be obtained by a reaction in which an azo-radical polymerization initiator having a low self-crosslinking reactivity is used as a radical polymerization initiator, and a chain transfer agent is added thereto during the polymerization reaction. In addition, the water-absorbent resin having a lowest water-retaining capacity (b) of physiological saline can be obtained by a reaction in which a radical polymerization initiator other than the azo-radical polymerization initiator is used, and a chain transfer agent and/or a metal is added thereto during the polymerization reaction.

The water-retaining capacity (b) of physiological saline of the water-absorbent resin is a value obtained in accordance with the determination method described in "(2) Water-Retaining Capacity of Physiological Saline" given later.

The water absorption (a) of physiological saline of the water-absorbent resin used in the present invention and the water-retaining capacity (b) of physiological saline of the water-absorbent resin used in the present invention satisfy the relationship of the formula:

$$[\text{Water Absorption (a) of Physiological Saline}] \geq [\text{Water-Retaining Capacity (b) of Physiological Saline}] + 15.$$

When the water absorption (a) of physiological saline and the water-retaining capacity (b) of physiological saline do not satisfy the relationship of the above formula, the water-absorbent resin is more likely to be formed into lumps, thereby lowering permeability and diffusibility. Therefore, when the water-absorbent resin is used as a thin absorbent, the amount of re-wet increases, thereby lowering the performance as the absorbent.

It is desired that the water absorption of physiological saline of the water-absorbent resin under pressure of 0.49 kPa is at least 55 g/g, preferably at least 60 g/g, more preferably at least 65 g/g, and even more preferably 70 to 100 g/g after 60 minutes passed from the beginning of water absorption from the viewpoint of being excellent in diffusibility of the aqueous liquid and obtaining high saturated water absorption capacity.

The water absorption of physiological saline of the water-absorbent resin under pressure of 0.49 kPa is a value obtained in accordance with the determination method described in "(3) Water Absorption of Physiological Saline Under Pressure of 0.49 kPa After 60 Minutes Passed from the Beginning of Water Absorption" given later.

In addition, when the water-absorbent resin begins absorbing water and the amount of water absorbed under pressure is small after a given time passed, the absorption amount of the absorbent under pressure becomes smaller. In other words, the absorbent absorbs a large amount of an aqueous liquid without impeding the diffusion of the aqueous liquid. On the other hand, when a load is applied to the absorbent during use of the absorbent article, the absorption amount of the absorbent would be smaller if the water absorption of the water-absorbent resin is smaller. Therefore, it is desired that the water absorption of physiological saline of the water-absorbent resin under pressure of 2.07 kPa after 60 minutes passed from the beginning of water absorption is at least 20 g/g, preferably at least 25 g/g, and more preferably 25 to 50 g/g.

The water absorption of physiological saline of the water-absorbent resin under pressure of 2.07 kPa is a value obtained in accordance with the determination method described in "(4) Water Absorption of Physiological Saline Under Pressure of 0.49 kPa After 60 Minutes Passed from the Beginning of Water Absorption" given later.

It is desired that the vortex time of the water-absorbent resin is within 100 seconds, preferably within 90 seconds, and more preferably within 80 seconds from the viewpoint of increasing a water absorption rate of the water-absorbent resin, thereby allowing the absorbent article to rapidly absorb the aqueous liquid to give a dry feel.

The vortex time of the water-absorbent resin is a value obtained in accordance with the determination method described in "(6) Vortex Time" given later.

It is desired that the water-absorbent resin has a weight-average particle diameter of at least 200 $\mu$m, preferably at least 250 $\mu$m from the viewpoint of lowering the amount of smaller size particles which are present, suppressing the worsening of handling of powder due to dusting or the like and suppressing the generation of gel blocking in the absorbent, which is a phenomenon in which swollen gels after absorption are contacted with each other to block a space between the water-absorbent resin particles. Also, it is desired that the water-absorbent resin has a weight-average particle diameter of at most 500 $\mu$m, and preferably at most 390 $\mu$m from the viewpoint of increasing diffusibility of the aqueous liquid, whereby the absorbent article can be comfortably used. From these viewpoints, it is desired that the water-absorbent resin has a weight-average particle diameter of 200 to 500 $\mu$m, and preferably 250 to 390 $\mu$m.

The weight-average particle diameter of the water-absorbent resin is a value obtained in accordance with the determination method described in "(5) Weight-Average Particle Diameter" given later.

The water-absorbent resin includes, for example, crosslinked polymers of acrylic acid salt, crosslinked vinyl alcohol-acrylic acid salt copolymers, crosslinked products of maleic anhydride-grafted polyvinyl alcohol, crosslinked isobutylene-maleic anhydride copolymers, crosslinked products of alkali salts of carboxymethyl cellulose, hydrolysates of starch-acrylonitrile graftcopolymers and the like. Among them, crosslinked polymers of acrylic acid salt are preferable. The water-absorbent resin can usually be obtained by polymerizing or copolymerizing a hydrophilic unsaturated monomer, and thereafter crosslinking the resulting polymer.

The hydrophilic unsaturated monomer is not limited to specified ones, and can be a hydrophilic unsaturated monomer which has been conventionally used for polymerization.

The hydrophilic unsaturated monomer includes, for example, monomers containing at least one kind selected from $\alpha,\beta$-unsaturated carboxylic acids such as acrylic acid, methacrylic acid, maleic acid and fumaric acid, and neutralized products thereof. Among them, acrylic acid, methacrylic acid and neutralized products thereof are preferable.

The hydrophilic unsaturated monomer may be copolymerized with other monomer as occasion demands. Other monomers include, for example, nonionic, hydrophilic group-containing monomers such as (meth)acrylamide ["(meth) acryl-" means "acryl-" and "methacryl-;" hereinafter referred to the same], N-substituted (meth)acrylamide, 2-hydroxyethyl (meth)acrylate, 2-hydroxypropyl (meth)acrylate, methoxypolyethylene glycol (meth)acrylate and polyethylene glycol (meth)acrylate; amino group-containing unsaturated monomers such as N,N-dimethylaminoethyl (meth)acrylate, N, N-dimethylaminopropyl (meth)acrylate and N,N-dimethylaminopropyl (meth)acrylamide; sulfonic acid-based monomers such as vinylsulfonic acid, styrenesulfonic acid, 2-(meth)acrylamide-2-methylpropanesulfonic acid, 2-(meth)acryloylethanesulfonic acid and salts thereof; and the like.

The hydrophilic unsaturated monomer is usually used in the state of an aqueous solution. It is preferable that the concentration of the hydrophilic unsaturated monomer in the aqueous solution of the hydrophilic unsaturated monomer is from 25% by weight to a saturated concentration.

It is desired that the degree of neutralization of the water-absorbent resin is 20 to 100% by mole, and preferably 30 to 90% by mole, based on the number of moles of the acidic groups in the water-absorbent resin. The neutralization can be applied to a starting monomer for the water-absorbent resin. Alternatively, the neutralization can be applied to the water-absorbent resin during polymerization or after polymerization.

A salt for neutralizing the water-absorbent resin includes, for example, alkali metal salts, alkaline earth metal salts, ammonium salts and the like, and salts of alkali metals such as sodium and potassium are preferable.

A polymerization method employed in the preparation of a water-absorbent resin is not limited to specified ones, and includes reverse phase suspension polymerization method, aqueous solution polymerization method, bulk polymerization method, precipitation polymerization method, and the like. Among these polymerization methods, reverse phase suspension polymerization method and aqueous solution polymerization method are preferable from the viewpoint of water absorbency and easy control of polymerization.

The reverse phase suspension polymerization method will be explained hereinbelow as an example of the method for polymerizing or copolymerizing a hydrophilic unsaturated monomer, without intending to limit the polymerization method thereto.

In the reverse phase suspension polymerization method, the polymerization is carried out, for example, by producing a dispersed state of an aqueous solution of the hydrophilic unsaturated monomer in an organic solvent, and polymerizing the monomer using, for example, a radical polymerization initiator in the presence of at least one of the surfactants and the polymeric protective colloids.

The organic solvent includes, for example, aliphatic hydrocarbon solvents such as n-pentane, n-hexane, n-heptane and ligroin; alicyclic hydrocarbon solvents such as cyclopentane, methylcyclopentane, cyclohexane and methylcyclohexane; aromatic hydrocarbon solvents such as benzene, toluene and xylene; and the like. Among these organic solvents, n-heptane and cyclohexane are preferable.

The surfactant includes, for example, nonionic surfactants such as sorbitan fatty acid esters, monoglycerol fatty acid esters, polyglycerol fatty acid esters, sucrose fatty acid esters, polyoxyethylene hydrogenated castor oil, polyoxyethylene lauric acid hydrogenated castor oil, polyoxyethylene (tri)isostearic acid hydrogenated castor oil, polyoxyethylene alkylphenyl ethers, polyoxyethylene lauryl ethers and polyoxyethylene hexyldecyl ethers; anionic surfactants such as salts of fatty acids, alkylbenzenesulfonic acid salts, alkyl methyl tauric acid salts, salts of polyoxyethylene alkylphenyl ether sulfuric esters, and polyoxyethylene alkyl ether sulfonic acid salts; and the like.

The polymeric protective colloid includes, for example, ethyl cellulose, ethylhydroxyethyl cellulose, polyethylene oxide, maleic anhydride-modified polyethylene, maleic anhydride-modified polybutadiene, maleic anhydride-modified ethylene-propylene-diene terpolymers, and the like.

These surfactants and polymeric protective colloids may be used in admixture of at least two kinds.

The amount of the surfactant and the polymeric protective colloid used is preferably 0.1 to 5% by weight, and more preferably 0.2 to 3% by weight based on the total amount of the hydrophilic unsaturated monomer. When the amount of the surfactant and the polymeric protective colloid used is less than 0.1% by weight, the hydrophilic unsaturated monomer is likely to be insufficiently dispersed. When the amount exceeds 5% by weight, the effect accounting to the amount used is less likely to be seen, and thereby being uneconomical.

When the polymerization is started, a radical polymerization initiator can be used. It is preferable to use as the radical polymerization initiator, for example, an azo-radical polymerization initiator such as 1,1'-azobis(cyclohexane-1-carbonitrile), 2,2'-azobis(2-methyl-butyronitrile), 2,2'-azobis(2,4-dimethylvaleronitrile), 2,2'-azobisisobutyronitrile, 2,2'-azobis(2-methylpropionamidino) dihydrochloride, 2,2'-azobis(2-amidinopropane) dihydrochloride, 4,4'-azobis-4-cyanovaleric acid, 2-cyano-2-propylazoformamide, dimethyl-2,2'-azobis(2-methylpropionate); a water-soluble radical polymerization initiator such as potassium persulfate, ammonium persulfate and sodium persulfate; and the like.

It is preferable that the amount of the radical polymerization initiator used is 0.005 to 1% by mole based on the total amount of the hydrophilic unsaturated monomer. When the amount of the radical polymerization initiator used is less than 0.005% by mole, the polymerization time is likely to be long. When the amount exceeds 1% by mole, the polymerization rapidly progresses, so that it is likely to be difficult to control the polymerization reaction.

The polymerization temperature during the polymerization reaction varies depending upon the kinds of the radical polymerization initiator and the hydrophilic unsaturated monomer used, the concentration of the aqueous monomer solution and the like. Usually, the polymerization temperature is preferably 20° to 110°C, and more preferably 40° to 90°C. When the polymerization temperature is lower than 20°C, the polymerization time is likely to be long, and when the polymerization temperature is higher than 110°C, it is difficult to remove the polymerization heat, so that it is more likely to be difficult to control the polymerization reaction. The reaction time is usually 0.1 to 4 hours.

Here, the water-absorbent resin may be subjected to internal crosslinking with an internal crosslinking agent having at least two polymerizable unsaturated groups or at least two reactive groups from the viewpoint of increasing water absorption and water-retaining capacity of the resulting water-absorbent resin. In addition, a chain transfer agent and/or a metal may be added during the polymerization reaction.

The internal crosslinking agent includes, for example, a compound having at least two unsaturated ethylenic groups in one molecule, such as N,N'-methylenebis(meth)acrylamide, ethylene glycol di(meth)acrylate, diethylene glycol di(meth)acrylate, triethylene glycol di(meth)acrylate, trimethylolpropane di(meth)acrylate, trimethylolpropane tri(meth)acrylate, trimethylolpropane di(meth)allyl ether and triallylamine; polyglycidyl ethers such as (poly)ethylene glycol diglycidyl ether and glycerol triglycidyl ether; and the like. These can be used alone or in admixture of at least two kinds, from the viewpoint of reactivity and water solubility in the polymerization system. Among the internal crosslinking agents, polyglycidyl ether is preferable.

The amount of the internal crosslinking agent used is at most 3% by weight, preferably at most 1% by weight, and more preferably at most 0.5% by weight based on the total amount of the hydrophilic unsaturated monomer. When the amount exceeds 3% by weight, the water absorption of the water-absorbent resin is likely to be lowered.

The chain transfer agent is not limited to specified ones, and includes ethanethiol, propanethiol, dodecanethiol, 1-butanethiol, thioglycolic acid, thiomalic acid, dimethyldithiocarbamic acid, diethyldithiocarbamic acid, L-cysteine, hypophosphorous acid, formic acid, and salts thereof; isopropanol; and the like.

The amount of the chain transfer agent used is 0.001 to 1% by mole, and preferably 0.005 to 0.3% by mole based on the total amount of the monomer. When the amount of the chain transfer agent used is less than 0.001% by mole, an effect of using the agent is less likely to be satisfactorily obtained, and when the amount exceeds 1% by mole, the gel strength is likely to be lowered.

The metal includes transition metals, typical metals belonging to Group 13 and typical metals belonging to Group 14. The transition metal includes, for example, titanium, zirconium, vanadium, niobium, chromium, molybdenum, tungsten, manganese, iron, cobalt, nickel, palladium, platinum, copper, silver, gold, zinc, cadmium, mercury and the like. The typical metal belonging to Group 13 includes, for example, alminium, gallium, and the like. The typical metal belonging to Group 14 includes, for example, tin, lead and the like. These metals may be used alone or a metal compound.

The metal compound includes, for example, oxalic acid salts, chlorides, perchloric acid salts, bromides, iodides, oxides, peroxides, carbonic acid salts, phosphides, fluorides, sulfides, nitric acid salts, sulfuric acid salts, sulfurous acid salts, nitrides, phosphinic acid salts, acetic acid salts, hydroxides, thiocyanic acid salts, ammonium sulfate, potassium sulfate, cyanides, azides, phosphoric acid salts, (ortho)silicic acid salts, silicides, chromic acid salts, borides, and the like. Among these metals, the element of iron or copper is preferable.

The amount of the metal used is 0.1 to 5% by millimole based on the total amount of the above-mentioned hydrophilic unsaturated monomer. When the amount of the metal used is less than 0.1% by millimole, an effect of using the metal is less likely to be satisfactorily obtained. In addition, when the amount exceeds 5% by millimole, the gel strength is likely to be lowered.

It is preferable that the water-absorbent resin is prepared by subjecting a hydrophilic unsaturated monomer to radical polymerization by the above-mentioned method or the like, and thereafter treating thus obtained polymer with a crosslinking agent having at least two functional groups having a reactivity with a carboxyl group to carry out post-crosslinking.

Post-crosslinking refers to crosslinking of a water-absorbent resin on its surface or into and near its surface, thereby increasing the crosslink density of a water-absorbent resin on its surface or into and near its surface as compared to that of the internal of the resin. A resin particle subjected to post-crosslinking forms a harder gel during absorption as compared to a product not being subjected to post-crosslinking. Therefore, there is less likely to cause gel blocking, a phenomenon in which a space between particles of the water-absorbent resin is blocked after absorption of the liquid, whereby the diffusibility of the liquid during absorption can be sufficiently maintained. Accordingly, when the resin particle is subjected to post-crosslinking, diffusion of liquid can be controlled at an earlier stage after the beginning of water absorption, and a saturated water absorption can be retained depending upon the hardness of the gel after a certain period of time passed from the beginning of the water absorption, whereby the amount of re-wet can be lowered.

As the post-crosslinking agent, those capable of reacting with carboxyl groups in the water-absorbent resin can be used. The post-crosslinking agent includes, for example, epoxy compounds such as (poly)ethylene glycol diglycidyl ether, (poly)propylene glycol diglycidyl ether, (poly)glycerol polyglycidyl ethers and glycidol; polyhydric alcohol compounds such as (poly)ethylene glycol, (poly)propylene glycol, (poly)glycerol, diols and trimethylolpropane; amine compounds such as diethanolamine and triethanolanime; and the like. These may be used alone or in combination of at least two kinds.

The amount of the post-crosslinking agent used is preferably 0.01 to 5% by weight, more preferably 0.02 to 4% by weight, and even more preferably 0.03 to 3% by weight based on the total amount of the above-mentioned monomer. When the amount of the post-crosslinking agent used is less than 0.01% by weight, the crosslink density of the portion near the surface of the water-absorbent resin cannot be sufficiently increased, so that the gel strength is likely to be lowered. When the amount exceeds 5% by weight, the crosslink density of the portion near the surface of the water-absorbent resin becomes too high so that water absorption is likely to be lowered.

Embodiments for adding the post-crosslinking agent are not limited to specified ones. It is preferable that the post-crosslinking agent is added to the water-absorbent resin after the post-crosslinking agent is dissolved in a hydrophilic solvent such as water in order to homogeneously disperse the post-crosslinking agent to the water-absorbent resin during the addition of the post-crosslinking agent. In addition, the above-mentioned post-crosslinking agent may be added in at least one divided portion.

The water-absorbent resin obtained by the polymerization method mentioned above is dried to remove water and an organic solvent therefrom, whereby a water-absorbent resin can be prepared. The resulting water-absorbent resin may be classified with sieves or the like as occasion demands.

A stabilizing agent can also be added to the water-absorbent resin thus obtained for the purpose of improving stability of the swollen gel during absorption of water. When the stabilizing agent is used, a physiologically active substance, such as a trace metal or vitamin C, which can be present in the absorbed liquid, is chemically inactivated, whereby lowering of water absorption of the swollen gel with the time passed can be prevented.

The stabilizing agent is not limited to specified ones, and includes, for example, reducing inorganic salts such as sodium sulfite, potassium sulfite and sodium hydrogensulfite; organic antioxidants such as gallic acid, propyl gallate, butylated hydroxyanisole and triphenyl phosphite; and the like.

The amount of the stabilizing agent used depends upon the kinds of the stabilizing agent. It is desired that the amount of the stabilizing agent used is 0.001 to 10 parts by weight, and preferably 0.01 to 5 parts by weight based on 100 parts by weight of the water-absorbent resin. A method for using the stabilizing agent includes, for example, a method comprising permeating a stabilizing agent into the internal of a water-absorbent resin; a method comprising adhering the stabilizing agent to its surface or into or near its surface of the water-absorbent resin; a method comprising mixing a stabilizing agent into a hydrophilic fiber, and homogeneously dispersing the stabilizing agent in an absorbent; a method comprising dispersing a layered stabilizing agent between a liquid-permeable sheet and an absorbent; and the like. Among these methods, the method comprising adhering a stabilizing agent to its surface or near its surface of the water-absorbent resin and a portion near its surface is more preferable in order to efficiently use the stabilizing agent.

EXAMPLES

The present invention will be explained hereinbelow by means of Examples, without intending to limit the present invention only to these Examples.

Preparation Example 1

The amount 340 g of n-heptane and 0.92 g of a sucrose fatty acid ester (manufactured by MITSUBISHI CHEMICAL CORPORATION under the trade name of S-370) were added to a 1000 mL-five-necked cylindrical round bottomed flask equipped with a stirrer, a reflux condenser, a dropping funnel, a thermometer and a nitrogen gas inlet tube. The mixture was dispersed in the flask, and the temperature of the dispersion was raised to dissolve the sucrose fatty acid ester, and thereafter the resulting solution was cooled to 55 °C.

Separately from the above, 92 g (1.02 mole) of an 80% by weight aqueous solution of acrylic acid was added to a 500 mL-Erlenmeyer flask. Thereto was added dropwise 102.2 g (0.76 mole) of a 30% by weight aqueous sodium hydroxide with cooling from external, to neutralize 75% by mole of acrylic acid. Further, 50.2 g of water, 0.11 g (0.41 mole) of a radical polymerization initiator potassium persulfate, and 9.2 mg of an internal crosslinking agent ethylene glycol diglycidyl ether were added thereto, to give an aqueous monomer solution for a first-step polymerization.

The entire amount of this aqueous monomer solution for a first-step polymerization was added to the above-mentioned five-necked cylindrical round bottomed flask with stirring, and the mixture was dispersed. After the internal of the system was sufficiently replaced with nitrogen, the temperature of the mixture was raised, and the polymerization reaction was carried out for 1 hour with keeping its bath temperature at 70°C. Thereafter, the polymerization slurry was cooled to room temperature.

The amount 119.1 g (1.32 mole) of an 80% by weight aqueous solution of acrylic acid was added to another 500 mL-Erlenmeyer flask. The amount 132.2 g (0.99 mole) of a 30% by weight aqueous sodium hydroxide solution was added dropwise thereto with cooling, to neutralize 75% by mole of acrylic acid. Further, 27.4 g of water, 0.14 g of a radical polymerization initiator potassium persulfate, and 35.7 mg of an internal crosslinking agent ethylene glycol diglycidyl ether were added thereto, to give an aqueous monomer solution for a second-step polymerization. The aqueous monomer solution was cooled in an ice water bath.

The entire amount of this aqueous monomer solution for a second-step polymerization was added to the above-mentioned polymerization slurry. After the internal of the system was again sufficiently replaced with nitrogen, the temperature of the mixture was raised, and the second-step polymerization reaction was carried out for 2 hours with keeping its bath temperature at 70°C. After the termination of the polymerization, the polymerization slurry was heated in an oil bath at 120°C, and the heated mixture was subjected to azeotropic distillation to remove water alone to external of the system, to give a gelated product. Subsequently, the amount 8.44 g of a 2% by weight aqueous solution of ethylene glycol diglycidyl ether was added to the resulting gelated product. Water and n-heptane were further removed from the mixture by distillation, and the residue was dried, to give 213.5 g of a water-absorbent resin (A).

Preparation Example 2

The same procedures as in Preparation Example 1 were carried out except that 2,2'-azobis(2-amidinopropane) dihydrochloride (manufactured by Wako Pure Chemical Industries, Ltd. under the product number of V-50) was used in place of the radical polymerization initiator potassium persulfate used in the aqueous monomer solutions for the first-step and second-step polymerizations, and that their amounts used were changed to 0.09 g (0.33 mmole) in the first step and 0.12 g (0.44 mmole) in the second step in Preparation Example 1, to give 212.4 g of a water-absorbent resin (B).

Preparation Example 3

The same procedures as in Preparation Example 1 were carried out except that the internal crosslinking agent ethylene glycol diglycidyl ether was not used in the aqueous monomer solution for a first-step polymerization, and that the amount of the internal crosslinking agent ethylene glycol diglycidyl ether used in the aqueous monomer solution for a second-step polymerization was changed from 35.7 mg to 11.9 mg in Preparation Example 1, to give 214.5 g of a water-absorbent resin (C).

Preparation Example 4

The amount 21.6 g of acrylic acid, 228.6 g of a 37% by weight sodium acrylate, 0.0925 g of a crosslinking agent N,N'-methylenebisacrylamide, and 53 g of ion-exchanged water were added to a 2000 mL-four-necked cylindrical round bottomed flask equipped with a stirrer, a reflux condenser, a dropping funnel, a thermometer, and a nitrogen gas inlet tube to give an aqueous monomer solution having a monomer concentration of 35% and a neutralization ratio of 75%. The amount 0.15 g of potassium persulfate was dissolved in this aqueous monomer solution, and a nitrogen gas was blown into the mixture to expel dissolved oxygen therefrom. Thereafter, the temperature of the mixture was raised, and the polymerization reaction was carried out for 1 hour with keeping its bath temperature at 65°C. After the termination of the polymerization, the polymerization slurry was heated in an oil bath at 120°C, and water was removed to external of the system, to give a gelated product. Subsequently, 4.22 g of a 2% by weight aqueous ethylene glycol diglycidyl ether solution was added to the resulting gelated product. Water was further removed from the mixture by distillation, and the residue was dried, to give 112.4 g of a water-absorbent resin (D).

Preparation Example 5

The same procedures as in Preparation Example 4 were carried out except that the internal crosslinking agent N,N'-methylenebisacrylamide in Preparation Example 4 was not used, to give 114.5 g of a water-absorbent resin (E).

Preparation Example 6

The same procedures as in Preparation Example 1 were carried out except that the internal crosslinking agent ethylene glycol diglycidyl ether was not used and 0.09 g (0.33 mmole) of 2,2'-azobis(2-amidinopropane)dihydrochloride (manufactured by Wako Pure Chemical Industries, Ltd. under the product number of V-50) was used in place of the radical polymerization initiator potassium persulfate in the aqueous monomer solution for a first-step polymerization, and that the amount of the internal crosslinking agent ethylene glycol diglycidyl ether used was changed from 35.7 mg to 11.9 mg and 0.12 g (0.44 mmole) of 2,2'-azobis(2-amidinopropane)dihydrochloride (manufactured by Wako Pure Chemical Industries, Ltd. under the product number of V-50) was used in place of the radical polymerization initiator potassium persulfate used in the aqueous monomer solution for a second-step polymerization in Preparation Example 1, to give 214.5 g of a water-absorbent resin (F).

Preparation Example 7

The same procedures as in Preparation Example 1 were carried out except that the internal crosslinking agent ethylene glycol diglycidyl ether was not used and 0.09 g (0.33 mmole) of 2,2'-azobis(2-amidinopropane)dihydrochloride (manufactured by Wako Pure Chemical Industries, Ltd. under the product number of V-50) was used in place of the radical polymerization initiator potassium persulfate in the aqueous monomer solution for a first-step polymerization, that the amount of the internal crosslinking agent ethylene glycol diglycidyl ether used was changed from 35.7 mg to 11.9 mg and 0.12 g (0.44 mmole) of 2,2'-azobis(2-amidinopropane)dihydrochloride (manufactured by Wako Pure Chemical Industries, Ltd. under the product number of V-50) was used in place of the radical polymerization initiator potassium persulfate used in the aqueous monomer solution for a second-step polymerization, and further that the amount of the 2% by weight aqueous ethylene glycol diglycidyl ether solution used to be added to the resulting gelated product was changed from 8.44 g to 12.66 g in Preparation Example 1, to give 215.2 g of a water-absorbent resin (G).

Preparation Example 8

The same procedures as in Preparation Example 1 were carried out except that to the aqueous monomer solutions for first-step and second-step polymerizations were added 0.028 g (0.31 mmole) of 1-butanethiol in the first step and 0.035 g (0.39 mmole) of 1-butanethiol in the second step in Preparation Example 1, to give 216.5 g of a water-absorbent resin (H).

Preparation Example 9

The same procedures as in Preparation Example 1 were carried out except that to the aqueous monomer solutions for first-step and second-step polymerizations were added 0.051 mg (0.0002 mmole) of iron in the first step and 0.066 mg (0.0011 mmole) of iron in the second step in Preparation Example 1, to give 212.5 g of a water-absorbent resin (I).

Preparation Example 10

The same procedures as in Preparation Example 1 were carried out except that 2,2'-azobis(2-amidinopropane) dihydrochloride (manufactured by Wako Pure Chemical Industries, Ltd. under the product number of V-50) was used in place of the radical polymerization initiator potassium persulfate used in the aqueous monomer solutions for first-step and second-step polymerizations, and that their amounts used were changed to 0.09 g (0.33 mmole) in the first step and to 0.12 g (0.44 mmole) in the second step in Preparation Example 1, to give 211.2 g of a water-absorbent resin. To the resulting water-absorbent resin was added 2.2 g of sodium sulfite with mixing, to give 213.4 g of a water-absorbent resin (J).

Preparation Example 11

The same procedures as in Preparation Example 1 were carried out except that 2,2'-azobis(2-amidinopropane) dihydrochloride (manufactured by Wako Pure Chemical Industries, Ltd. under the product number of V-50) was used in place of the radical polymerization initiator potassium persulfate used in the aqueous monomer solutions for first-step and second-step polymerizations, that their amounts used were changed to 0.09 g (0.33 mmole) in the first step and to 0.12 g (0.44 mmole) in the second step to give a gelated product, and that the amount of the 2% by weight aqueous ethylene glycol diglycidyl ether solution used to be added to the resulting gelated product was changed from 8.44 g to 16.88 g in Preparation Example 1, to give 218.2 g of a water-absorbent resin (K).

Preparation Example 12

The same procedures as in Preparation Example 1 were carried out except that the 2% by weight aqueous ethylene glycol diglycidyl ether solution to be added to the resulting gelated product was not used in Preparation Example 1, to give 218.2 g of a water-absorbent resin (L).
Next, the resulting water-absorbent resin was evaluated in accordance with the following methods. The results are shown in Table 1.

(1) Water Absorption of Physiological Saline

In a 1000 mL beaker, 2 g of a water-absorbent resin was dispersed in 1000 g of physiological saline (0.9% by weight aqueous sodium chloride), and the dispersion was gently stirred for 1 hour to sufficiently swell the resin. On the other hand, the physiological saline containing the swollen gel was filtered with a JIS standard sieve having an opening of 75 $\mu$m, the JIS standard sieve of which weight Wa (g) was previously determined. The filtered sieve was allowed to stand for 30 minutes in a state so that the sieve was tilted at a tilt angle of about 30 degrees to the horizontal to remove excess physiological saline from the water-absorbent resin. After a weight Wb (g) of the sieve containing the swollen gel was determined, the water absorption was calculated in accordance with the following formula:

$$[\text{Water Absorption of Physiological Saline (g/g)}] = (Wb - Wa)/2$$

(2) Water-Retaining Capacity of Physiological Saline

Two grams of a water-absorbent resin was placed in a cotton bag (Cottonbroad No. 60, width 100 mm × length 200 mm), and the cotton bag was placed in a 500 mL-beaker. Physiological saline was poured into the cotton bag in an amount of 500 g at a time, and the saline was dispersed so as not to generate a lump of the water-absorbent resin. The upper part of the cotton bag was tied up with a rubber band, and the cotton bag was allowed to stand for 1 hour, to sufficiently swell the water-absorbent resin. The cotton bag was spin-dried for 1 minute with a spin dryer (manufactured by Kokusan Enshinki Co., Ltd., H-122) set to have a centrifugal force of 167G, and a weight Wc (g) of the cotton bag containing swollen gels after the dehydration was determined. The same procedures were carried out without adding a water-absorbent resin, and an empty weight Wd (g) of the cotton bag upon wetting was determined. The water-retaining capacity was calculated in accordance with the following formula:

$$[\text{Water-Retaining Capacity of Physiological Saline (g/g)}] = (Wc - Wd)/2$$

(3) Water Absorption of Physiological Saline under Pressure of 0.49 kPa After 60 Minutes Passed from the Beginning of Water Absorption

The amount of water absorption of physiological saline under pressure of 490 Pa after 60 minutes passed from the beginning of water absorption was determined using a measuring apparatus X shown in Figure 1.
The measuring apparatus X shown in Figure 1 comprises an electronic balance 1, a bottle 2 placed on the electronic balance 1, an air aspiration tube 3, a lead pipe 4, a glass filter 5, and a measuring section 6 placed on the glass filter 5. The electronic balance 1 is connected to a computer 7 so that change in weight can be recorded in the units of seconds or minutes. The bottle 2 holds physiological saline in the internal thereof, and the air aspiration tube 3 is inserted in an opening on its top, and the lead pipe 4 is attached to the body section.
The lower end of the air aspiration tube 3 is soaked in the physiological saline 8. The glass filter 5 has a diameter of 25 mm. As the glass filter 5, Glass Filter No. 1 of Sogo Rikagaku Glass Seisakusho (pore diameter: 100 to 160 $\mu$m) was used. The bottle 2 and the glass filter 5 are communicated with each other via the lead pipe 4. In addition, the glass filter 5 is fixed to a position slightly higher than the lower end of the air aspiration tube 3.
The measuring section 6 has a cylinder 60, a nylon mesh 61 adhered to the bottom part of the cylinder 60, and a weight 62. The cylinder 60 has an inner diameter of 20 mm. The nylon mesh 61 is formed to have a size of 200 mesh screen (size of opening: 75 $\mu$m). The weight 62 has a diameter of 19 mm and a weight of 14.2 g. A given amount of a water-absorbent resin 9 is evenly spread over the nylon mesh 61. The weight 62 is placed on the water-absorbent resin 9, so that a 0.49 kPa load can be applied to the water-absorbent resin 9.
In the measuring apparatus X having the constitution as described above, first, the bottle 2 is charged with physiological saline 8 in a given amount, and the air aspiration tube 3 is placed in the bottle 2 to get ready for the determination. Next, 0.1 g of the water-absorbent resin 9 was evenly spread over the nylon mesh 61 in the cylinder 60, and the weight 62 was placed on the water-absorbent resin 9. The measuring section 6 was placed on the glass filter 5 so that its central section was in alignment with the central section of the glass filter 5.

On the other hand, the computer 7 connected to the electronic balance 1 was booted, and the weight reduction of the physiological saline in the bottle 2, i.e., the weight of the physiological saline absorbed by the water-absorbent resin 9, We (g), was continuously recorded on the computer 7 from the time when the water-absorbent resin 9 started absorbing water, in units of minutes and preferably in units of seconds on the basis of the value obtained from the electronic balance 1. The amount of water absorption of physiological saline under pressure of 0.49 kPa after 60 minutes passed from the beginning of the water absorption was calculated by dividing the weight We (g) after 60 minutes passed by the weight of the water-absorbent resin 9 (0.1 g).

(4) Water Absorption of Physiological Saline Under Pressure of 2.07 kPa After 60 Minutes Passed from the Beginning of Water Absorption

The same procedures for the determination as in the above (3) were carried out except that the weight of the weight 62 was changed from 14.2 g to 59.8 g in the determination mentioned in the above (3), to calculate the water absorption of physiological saline under pressure of 2.07 kPa after 60 minutes passed from the beginning of water absorption.

(5) Weight-Average Particle Diameter

One-hundred grams of a water-absorbent resin was weighed and placed on an uppermost sieve of 8 standard sieves complying with JIS-Z 8801-1982, in which the sieves having openings of 850 $\mu$m, 500 $\mu$m, 355 $\mu$m, 300 $\mu$m, 250 $\mu$m, 180 $\mu$m, 106 $\mu$m and the bottom of the container were stacked in order from the top, and shaken with a rotating and tapping shaker machine for 10 minutes to sieve the water-absorbent resin, and thereafter the water-absorbent resin remaining on each sieve was weighed. In accordance with the results, the particle diameter of which cumulative weight is 50% was calculated in accordance with the following formula:

$$\text{[Weight-Average Particle Diameter } (\mu m)] = [(50\text{-}A)/(D\text{-}A)] \times (C - B) + B$$

wherein A is the cumulative value (g) obtained when the weights are sequentially accumulated from those having coarser particle diameters, to obtain a cumulative value of which cumulative weight is less than 50% by weight and most closely approximating 50% by weight; and B is an opening ($\mu$m) of the sieve at which the cumulative value is obtained.
In addition, D is the accumulated value obtained when the weights are sequentially accumulated from those having coarse particle diameters to obtain a cumulative value of which cumulative weight is at least 50% by weight and most closely approximating 50% by weight; and C is an opening ($\mu$m) of the sieve at which the cumulative value is obtained.

(6) Vortex Time

The amount 50 $\pm$ 0.01 g of physiological saline adjusted to the range of 25° $\pm$ 1°C was weighed out in a 100 mL beaker. A magnetic stirrer bar having a size of 8 mm $\times$ 30 mm without a ring was placed in the beaker, and the beaker was placed on the top of MAGNETIC STIRRER (manufactured by IUCHI, HS-30D). Subsequently, the magnetic stirrer bar was adjusted so that the magnetic stirrer bar rotated at 600 ppm, and further adjusted so that the bottom of the vortex generated by the rotation of the magnetic stirrer bar came near the upper portion of the magnetic stirrer bar.
Next, in the determination of "(5) Weight-Average Particle Diameter" mentioned above, 1.0 $\pm$ 0.002 g of the mixture of the water absorbent resins remaining on sieves having openings of 355 $\mu$m and 300 $\mu$m was quickly poured between the center of vortex in the beaker and the side of the beaker, and the time (seconds) from a point where the water-absorbent resin was poured into the beaker to a point where the vortex converged was determined with a stopwatch.

Table 1

| Prep. Ex. No. | Physiological Saline | | Water Absorption of Physiological Saline Under Pressure (g/g) | | Weight-Average Particle Diameter (μm) | Vortex Time (sec) |
|---|---|---|---|---|---|---|
| | Water Absorption (g/g) | Water-Retaining Capacity (g/g) | Under Pressure of 0.49 kPa | Under Pressure of 2.07 kPa | | |
| 1 | 62 | 41 | 45 | 32 | 367 | 114 |
| 2 | 83 | 63 | 65 | 37 | 355 | 77 |
| 3 | 72 | 56 | 57 | 35 | 386 | 75 |
| 4 | 55 | 35 | 42 | 30 | 399 | 153 |
| 5 | 72 | 52 | 71 | 32 | 322 | 95 |
| 6 | 97 | 77 | 79 | 32 | 344 | 88 |
| 7 | 81 | 61 | 66 | 34 | 335 | 79 |
| 8 | 94 | 74 | 69 | 30 | 398 | 82 |
| 9 | 98 | 78 | 77 | 28 | 387 | 89 |
| 10 | 77 | 57 | 58 | 35 | 378 | 77 |
| 11 | 50 | 30 | 39 | 31 | 377 | 137 |
| 12 | 76 | 73 | 51 | 10 | 399 | 125 |

Example 1

A mixture prepared by dry-blending 6 g of the water-absorbent resin (B) obtained in Preparation Example 2 and 10 g of pulverized pulp with a mixer was sprayed on tissue paper having a size of 40 cm × 10 cm and a weight of 1 g. Thereafter, tissue paper having the same size and the same weight as above was layered over the top, and formed into a sheet. A 196 kPa load was applied to the entire sheet for 30 seconds to press the sheet, thereby giving an absorbent having a concentration of a water-absorbent resin of 37.5% by weight. The resulting absorbent was interposed between a polyethylene air-through, porous liquid-permeable sheet having a size of 40 cm × 12 cm and a basis weight of 20 g/cm$^2$ and a polyethylene impermeable sheet having the same size and the same basis weight as above to give an absorbent article using the absorbent having a concentration of a water-absorbent resin of 37.5% by weight. Similarly, 10 g of the water-absorbent resin (B) and 10 g of pulverized pulp were used to give an absorbent having a concentration of a water-absorbent resin of 50% by weight and an absorbent article using the absorbent.

Example 2

The same procedures as in Example 1 were carried out except that the water-absorbent resin (C) obtained in Preparation Example 3 was used, in Example 1, to give absorbents having concentrations of a water-absorbent resin of 37.5% by weight and 50% by weight, and an absorbent article using each of the absorbents.

Example 3

The same procedures as in Example 1 were carried out except that the water-absorbent resin (E) obtained in Preparation Example 5 was used, in Example 1, to give absorbents having concentrations of a water-absorbent resin of 37.5% by weight and 50% by weight, and an absorbent article using each of the absorbents.

Example 4

The same procedures as in Example 1 were carried out except that the water-absorbent resin (F) obtained in Preparation Example 6 was used, in Example 1, to give absorbents having concentrations of a water-absorbent resin of 37.5% by weight and 50% by weight, and an absorbent article using each of the absorbents.

Example 5

The same procedures as in Example 1 were carried out except that the water-absorbent resin (G) obtained in Preparation Example 7 was used, in Example 1, to give absorbents having concentrations of a water-absorbent resin of 37.5% by weight and 50% by weight, and an absorbent article using each of the absorbents.

Example 6

The same procedures as in Example 1 were carried out except that the water-absorbent resin (H) obtained in Preparation Example 8 was used, in Example 1, to give absorbents having concentrations of a water-absorbent resin of 37.5% by weight and 50% by weight, and an absorbent article using each of the absorbents.

Example 7

The same procedures as in Example 1 were carried out except that the water-absorbent resin (I) obtained in Preparation Example 9 was used, in Example 1, to give absorbents having concentrations of a water-absorbent resin of 37.5% by weight and 50% by weight, and an absorbent article using each of the absorbents.

Example 8

The same procedures as in Example 1 were carried out except that the water-absorbent resin (J) obtained in Preparation Example 10 was used, in Example 1, to give absorbents having concentrations of a water-absorbent resin of 37.5% by weight and 50% by weight, and an absorbent article using each of the absorbents.

Comparative Example 1

The same procedures as in Example 1 were carried out except that the water-absorbent resin (A) obtained in Preparation Example 1 was used, in Example 1, to give absorbents having concentrations of a water-absorbent resin of 37.5% by weight and 50% by weight, and an absorbent article using each of the absorbents.

Comparative Example 2

The same procedures as in Example 1 were carried out except that the water-absorbent resin (D) obtained in Preparation Example 4 was used, in Example 1, to give absorbents having concentrations of a water-absorbent resin of 37.5% by weight and 50% by weight, and an absorbent article using each of the absorbents.

Comparative Example 3

The same procedures as in Example 1 were carried out except that the water-absorbent resin (K) obtained in Preparation Example 11 was used, in Example 1, to give absorbents having concentrations of a water-absorbent resin of 37.5% by weight and 50% by weight, and an absorbent article using each of the absorbents.

Comparative Example 4

The same procedures as in Example 1 were carried out except that the water-absorbent resin (L) obtained in Preparation Example 12 was used, in Example 1, to give absorbents having concentrations of a water-absorbent resin of 37.5% by weight and 50% by weight, and an absorbent article using each of the absorbents.

Reference Example

A mixture prepared by dry-blending 18 g of the water-absorbent resin (K) obtained in Preparation Example 11 and 10 g of pulverized pulp with a mixer was sprayed to tissue paper having a size of 40 cm $\times$ 10 cm and a weight of 1 g. Thereafter, tissue paper having the same size and the same weight as above was layered over the top, and formed into a sheet. A 196 kPa load was applied to the entire sheet for 30 seconds to press the sheet, thereby giving an absorbent having a concentration of a water-absorbent resin of 64.3% by weight. The resulting absorbent was interposed between a polyethylene air-through, porous liquid-permeable sheet having a size of 40 cm $\times$ 12 cm and a basis weight of 20 g/cm$^2$ and a polyethylene impermeable sheet having the same size and the same basis weight as above, to give an absorbent article using the absorbent having a concentration of a water-absorbent resin of 64.3% by weight.

Next, the resulting absorbent article was evaluated in accordance with the following method. The results of the absorbent article using the absorbent having a concentration of a water-absorbent resin of 37.5% by weight are shown in Table 2, and the results of the absorbent article using the absorbent having a concentration of a water-absorbent resin of 50% by weight and the absorbent article obtained in Reference Example are shown in Table 3.

(a) Preparation of Artificial Urine

Sixty grams of sodium chloride, 1.8 g of calcium chloride dihydrate, 3.6 g of magnesium chloride hexahydrate, and a proper amount of distilled water were placed in a 10 L container, and were completely dissolved. Next, 0.02 g of polyoxyethylene nonyl phenyl ether was added to the mixture, and distilled water was further added thereto to make up a weight of 6000 g of the entire aqueous solution. Further, the aqueous solution was colored with a small amount of Blue No. 1, to give an artificial urine.

(b) Permeation Time

A cylinder having an inner diameter of 3 cm was placed near the center of the absorbent article, and 50 mL of the artificial urine which was previously kept at 25 ± 0.2°C in a thermostatic water tank was poured therein. At the same time, a stopwatch was clicked to start to determine the time required until the artificial urine was completely permeated into the absorbent article (first time). Next, the above-mentioned cylinder was removed and the absorbent article was kept in that state. After 30 minutes passed from the beginning of the first pouring of the artificial urine, the above-mentioned cylinder was again placed on the same position as before, and 50 mL of the artificial urine was poured therein. At the same time, the stopwatch was clicked to start to determine the time required until the artificial urine was completely permeated in the absorbent article (second time). Further, the permeation time was determined up to three times for an absorbent article using the absorbent having a water-absorbent resin concentration of 37.5% by weight, and five times for an absorbent article using the absorbent having a water-absorbent resin concentration of 50% by weight and the absorbent article obtained in Reference Example in the same manner as above.

(c) Amount of Re-wet

After 60 minutes passed from the final determination of the above-mentioned permeation time, filter papers (Toyo Roshi Kaisha , Ltd., No. 2), which was cut into pieces having a size of 10 cm × 10 cm, were stacked in layers to obtain an about 80 g portion of filter papers, and its dry weight (g) was determined. The filter paper was placed on the central part of the absorbent article, and a 5 kg weight (base area = 10 cm × 10 cm) was placed thereon to apply a load for 5 minutes. Thereafter, the weight was removed therefrom and the weight (g) of the filter paper absorbing the re-wet liquid was determined. The amount of re-wet was calculated by subtracting the weight (g) of the dry filter paper from the weight (g) of the filter paper absorbing the re-wet liquid.

(d) Diffusion Length

The diffusion size (cm) in the longitudinal direction of each absorbent article in which the artificial urine was permeated was determined within 5 minutes after the determination of the above-mentioned amount of re-wet. Here, the numerical values of decimal places were rounded to the nearest whole number.

Table 2

| Absorbent Article Using Absorbent Having Concentration of Water-Absorbent Resin of 37.5% by Weight | | | | | | |
|---|---|---|---|---|---|---|
| Ex. No. | Permeation Time (sec) | | | | Amount of Re-wet (g) | Diffusion Length (cm) |
| | First | Second | Third | Total | | |
| Ex.1 | 32 | 31 | 35 | 98 | 24.4 | 29 |
| 2 | 29 | 29 | 35 | 93 | 29.2 | 30 |
| 3 | 27 | 32 | 40 | 99 | 35.0 | 27 |
| 4 | 34 | 34 | 36 | 104 | 17.0 | 27 |
| 5 | 32 | 30 | 33 | 95 | 25.5 | 30 |
| 6 | 35 | 32 | 36 | 103 | 22.0 | 28 |
| 7 | 36 | 33 | 37 | 106 | 19.0 | 29 |
| 8 | 31 | 32 | 34 | 97 | 22.0 | 28 |
| Comp. Ex. 1 | 30 | 27 | 38 | 95 | 48.0 | 28 |
| 2 | 27 | 35 | 42 | 104 | 65.9 | 31 |
| 3 | 31 | 25 | 40 | 96 | 54.7 | 30 |
| 4 | 33 | 30 | 48 | 111 | 26.9 | 26 |

Table 3

| Absorbent Article Using an Absorbent Having a Water-Absorbent Resin Concentration of 50% by Weight | | | | | | | | |
|---|---|---|---|---|---|---|---|---|
| Ex. No. | Permeation Time (sec) | | | | | | Amount of Re-wet (g) | Diffusion Length (cm) |
| | First | Second | Third | Fourth | Fifth | Total | | |
| Ex.1 | 22 | 20 | 23 | 26 | 34 | 125 | 28.7 | 22 |
| 2 | 21 | 19 | 24 | 28 | 35 | 127 | 30.0 | 23 |
| 3 | 24 | 22 | 25 | 28 | 32 | 131 | 22.9 | 21 |
| 4 | 24 | 21 | 21 | 27 | 33 | 126 | 10.5 | 20 |
| 5 | 23 | 21 | 21 | 26 | 33 | 124 | 19.2 | 23 |
| 6 | 25 | 23 | 24 | 25 | 34 | 131 | 17.8 | 24 |
| 7 | 27 | 20 | 25 | 30 | 34 | 136 | 12.8 | 22 |
| 8 | 22 | 22 | 24 | 27 | 34 | 129 | 14.0 | 23 |
| Comp. Ex. 1 | 20 | 17 | 21 | 27 | 39 | 124 | 47.3 | 24 |
| 2 | 19 | 15 | 18 | 28 | 49 | 129 | 61.5 | 27 |
| 3 | 20 | 18 | 22 | 26 | 37 | 123 | 48.2 | 24 |
| 4 | 21 | 20 | 24 | 28 | 50 | 143 | 22.6 | 22 |
| Ref. Ex. | 21 | 18 | 22 | 28 | 37 | 126 | 14.2 | 23 |

It can be seen from the results shown in Tables 2 and 3 that the absorbents and the absorbent articles using the absorbents in Examples 1 to 8 have small amounts of re-wet, high permeation rates, and large diffusion lengths, without increasing the amount of the water-absorbent resin in the absorbent.

INDUSTRIAL APPLICABILITY

According to the present invention, an absorbent having a small amount of re-wet of an aqueous liquid, a high permeation rate, and being excellent in diffusibility, without increasing the amount of the water-absorbent resin in the absorbent, and an absorbent article using the absorbent can be provided. Accordingly, thinning and light weight of the absorbent and the absorbent article can be achieved.

**Claims**

1. An absorbent comprising a water-absorbent resin and a hydrophilic fiber, **characterized in that** said water-absorbent resin has a water absorption (a) of physiological saline of 60 to 100 g/g and a water-retaining capacity (b) of physiological saline of 45 to 80 g/g, and that the water absorption (a) of physiological saline and the water-retaining capacity (b) of physiological saline satisfy the relationship of the formula:

$$[\text{Water absorption (a) of physiological saline}] \geq [\text{Water-retaining capacity (b) of physiological saline}] + 15.$$

2. The absorbent according to claim 1, wherein the water-absorbent resin has a water absorption of physiological saline under pressure of 0.49 kPa of at least 55 g/g after 60 minutes passed from the beginning of water absorption.

3. The absorbent according to claim 1 or 2, wherein the water-absorbent resin has a water absorption of physiological saline under pressure of 2.07 kPa of at least 20 g/g after 60 minutes passed from the beginning of water absorption.

4. The absorbent according to any one of claims 1 to 3, wherein the vortex time of the water-absorbent resin is within 100 seconds.

5. An absorbent article comprising the absorbent according to any one of claims 1 to 4 interposed between a liquid-permeable sheet and a liquid-impermeable sheet.

# FIG. 1

<div align="center">

**INTERNATIONAL SEARCH REPORT**

</div>

| International application No. |
| --- |
| PCT/JP2004/008377 |

**A. CLASSIFICATION OF SUBJECT MATTER**
Int.Cl⁷ A61F13/53, A61L15/60, B01J20/26

According to International Patent Classification (IPC) or to both national classification and IPC

**B. FIELDS SEARCHED**

Minimum documentation searched (classification system followed by classification symbols)
Int.Cl⁷ A61F13/15-53, A61L15/60, B01J20/26

Documentation searched other than minimum documentation to the extent that such documents are included in the fields searched

| | | | |
| --- | --- | --- | --- |
| Jitsuyo Shinan Koho | 1926-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 8-57311 A (Nippon Shokubai Co., Ltd.), 05 March, 1996 (05.03.96), Claims; Par. Nos. [0028] to [0035], [0056] to [0064] & WO 95/034377 A & EP 712659 A | 1-5 |
| Y | JP 2003-88551 A (Sumitomo Seika Chemicals Co., Ltd.), 25 March, 2003 (25.03.03), Claims; Par. Nos. [0025], [0030] to [0056] (Family: none) | 1-5 |
| Y | JP 6-254118 A (Kimberly-Clark Corp.), 13 September, 1994 (13.09.94), Tables 4, 5 & EP 615736 A & US 5601542 A | 4 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 06 July, 2004 (06.07.04) | 20 July, 2004 (20.07.04) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

## INTERNATIONAL SEARCH REPORT

| International application No. |
|---|
| PCT/JP2004/008377 |

C (Continuation). DOCUMENTS CONSIDERED TO BE RELEVANT

| Category* | Citation of document, with indication, where appropriate, of the relevant passages | Relevant to claim No. |
|---|---|---|
| A | JP 9-276391 A  (Kao Corp.),<br>28 October, 1997 (28.10.97),<br>Claims; Par. Nos. [0021], [0028], [0043]<br>(Family: none) | 1-5 |
| A | JP 2003-88553 A  (Sumitomo Seika Chemicals Co., Ltd.),<br>25 March, 2003 (25.03.03),<br>Claims; Par. Nos. [0034] to [0064]<br>(Family: none) | 1-5 |
| A | JP 7-88171 A  (Sanyo Chemical Industries, Ltd.),<br>04 April, 1995 (04.04.95),<br>& EP 629411 A | 1-5 |

Form PCT/ISA/210 (continuation of second sheet) (January 2004)